# EUROPÄISCHE PATENTANMELDUNG

(11) **EP 1 623 735 A1**
(43) Veröffentlichungstag der Anmeldung: **08.02.2006**
(21) Anmeldenummer: 04018532.4
(22) Anmeldetag: 05.08.2004
(51) Int. Cl.: A61M 5/168, F16K 1/12, F16K 27/02

(54) **Durchflussregelventil**

(71) Anmelder: Gemü GmbH, 6343 Rotkreuz (CH)
(72) Erfinder: Rominger, Lars, 6313 Edlibach (CH)
(74) Vertreter: Troesch Scheidegger Werner AG

(57) **Zusammenfassung**

Eine Dosiervorrichtung für fluide Medien weist mindestens zwei in Richtung zueinander bzw. voneinander bewegbare Teile (1, 11) auf. Die beiden Teile weisen je einen flächigen Abschnitt (5, 15) auf, welche voneinander beabstandet benachbart zueinander angeordnet sind, derart, dass beim Bewegen der beiden Teile der Abstand (12) zwischen den beiden flächigen Abschnitten verkleinerbar bzw. vergrösserbar ist, und dass der Durchfluss des fluiden Mediums zwischen mindestens an Teilbereichen der beiden Abschnitten bzw. Flächen regulierbar bzw. dosierbar ist.

## Beschreibung

Die vorliegende Erfindung betrifft eine PräzisionsDosiervorrichtung für fluide Medien gemäss dem Oberbegriff nach Anspruch 1, ein Verfahren zum Dosieren eines fluiden Mediums sowie eine Verwendung der Vorrichtung.

Bei Infusionen, an Kathetern, etc. werden in der Regel sowohl Tropfenzähler wie auch sogenannte Quetschventile verwendet um einerseits eine visuelle Prüfung der Infusion und andererseits eine Dosierung zu ermöglichen.

Die verwendeten Quetschventile sind relativ ungenau in der Dosierung und zudem sind Fehlmanipulationen möglich. Auch wäre es vorteilhaft statt der beiden geschilderten, separaten Elemente ein einziges Organ für beide Funktionen einsetzen zu können.

Es ist deshalb eine Aufgabe der vorliegenden Erfindung eine Alternative zu den heute gebräuchlichen Quetschventilen bzw. Dosiervorrichtungen, in Fluidiksystemen, wie insbesondere bei Infusionen, Medikamentverabreichung, Spülungen, etc., zu schaffen.

Erfindungsgemäss wird eine Dosiervorrichtung, wie insbesondere ein sogenanntes Präzisions-Dosimeter, für fluide Medien gemäss dem Wortlaut nach Anspruch 1 vorgeschlagen.

Die erfindungsgemäss vorgeschlagene Dosiervorrichtung weist mindestens zwei in Richtung zueinander bzw. voneinander bewegbare Teile auf, wobei die beiden Teile je einen flächigen Abschnitt aufweisen, welche Abschnitte zueinander benachbart angeordnet und voneinander beabstandet sind derart, dass beim Bewegen der beiden Teile der Abstand zwischen den beiden flächigen Abschnitten verkleinert bzw. vergrössert wird und der Durchfluss des fluiden Mediums geführt mindestens zwischen Teilbereichen der beiden flächigen Abschnitten regulierbar bzw. dosierbar ist.

Gemäss einer Ausführungsvariante sind die beiden Flächen bzw. flächigen Abschnitte wenigstens entlang eines Teilbereiches zueinander wenigstens nahezu formschlüssig passend ausgebildet.

Die beiden Teile sind beispielsweise mittels eines Schraubgewindes voneinander bzw. zueinander bewegbar, indem beispielsweise am einen Teil eine Gewindekontur ausgebildet ist, welche dazu vorgesehen ist, um in einem entsprechenden Gewinde am anderen Teil eingreifend drehbar bewegt zu werden, um so den Abstand der beiden Flächen zu vergrössern bzw. zu verkleinern.

Gemäss einer Ausführungsvariante ist das eine Teil resp. die entsprechende Oberfläche kegelförmig ausgebildet und das andere Teil bzw. dessen Oberfläche wenigstens nahezu dazu entsprechend hohlkegelförmig. Im oder am einen Teil aufweisend die Kegelform ist ein Durchgang für das fluide Medium vorgesehen, damit das fluide Medium in den Zwischenraum zwischen die beiden Teile bzw. deren zueinander gerichteten Oberflächen einfliessen kann.

Im anderen hohlkegelförmig ausgebildeten Teil aufweisend die entsprechend eine hohlkegelförmig Oberfläche, ist wiederum ein Durchgang bzw. ein Abfluss vorgesehen, durch welchen das fluide Medium den Zwischenraum verlassen kann.

Ist der Abstand zwischen den beiden flächigen Abschnitten der beiden Teile verhältnismässig gross, kann eine grössere Menge des fluiden Mediums durch die Dosiervorrichtung hindurch gelangen, währenddem bei kleinem resp. minimalstem Abstand zwischen den beiden Flächen bzw. bei kleinstem Spalt nur eine sehr kleine Menge des fluiden Mediums durch die Dosiervorrichtung hindurch gelangen kann. Falls die beiden Flächen vollständig aneinander anliegen ist die Dosiervorrichtung verschlossen.

Selbstverständlich handelt es sich bei der kegelförmigen Oberfläche und der entsprechend ausgebildeten hohlkegelförmig ausgebildeten Oberfläche nur um ein Beispiel und auch andere Flächendesigns können gewählt werden, wie beispielsweise eine halbkugelförmig ausgebildet Oberfläche und eine entsprechend dazu hohlkugelförmig ausgebildete Fläche. Wiederum andere Ausbildungen der Flächen sind möglich, bevorzugt werden die beiden Flächen so gewählt, dass einerseits ein weitgehendst formschlüssiges Aneinanderfügen der beiden Flächen mindestens in einem Teilbereich möglich ist, derart, dass in diesem Bereich die Dosierung des fluiden Mediums möglich ist.

Weiter wird vorgeschlagen, am einen Teil, im Sinne eines Tropfenzählers, eine Zuflusstropfkammer vorzusehen. Diese Tropfkammer kann entweder integral mit dem einen Teil verbunden sein, oder aber, ebenfalls am einen Teil aufgesteckt oder aufgeschraubt werden. An der Zuflusstropfkammer ist vorzugsweise ein Anschluss vorgesehen zum Anschliessen der Tropfkammer beispielsweise an einem Schlauch, wie an einem Katheter.

Entsprechend ist am anderen Teil ebenfalls ein Anschluss vorgesehen, um das andere Teil wiederum an einem Schlauch, wie beispielsweise an einem Katheter, anzuschliessen.

Entsprechend der Vorrichtung wird ein Verfahren zum Dosieren eines fluiden Mediums, beispielsweise bei Infusionen und dgl., gemäss dem Wortlaut nach Anspruch 7 vorgeschlagen. Vorgeschlagen wird, dass das fluide Medium in einen Zwischenraum zwischen zwei voneinander beabstandete Flächen von je einem Teil einer Dosiervorrichtung eingebracht wird, und dass durch Bewegen der beiden Teile zueinander bzw. voneinander der Zwischenraum bzw. der Abstand der beiden Flächen verkleinert bzw. vergrössert wird, um das fluide Medium zu dosieren.

Weitere bevorzugte Ausführungsvarianten sowohl der Dosiervorrichtung wie auch des Verfahrens sind in den abhängigen Ansprüchen charakterisiert.

Sowohl die oben erwähnte Dosiervorrichtung, wie beispielsweise ein Präzisions-Dosimeter, wie auch das beschriebene Verfahren, sind insbesondere geeignet:
- Für alle Druck- und Schwerkraftinfusionen mit und ohne integriertem Rückschlagventil zur Konnektion mit Infusionssystemen, sowie mit und ohne Belüftung.
- Enterale Ernährung, Enterale Medikamenten- und Flüssigkeitsverabreichung.
- Rektale Intra- und postoperative Blasenspülung (Urologie).
- Für alle Druck- und Schwerkraftinfusionen.
- Sowie alle weiteren Anwendungen die den Zweck haben Flüssigkeiten zu transportieren und die hier nicht abschliessend aufgeführt sind.

Die Erfindung wird nun beispielsweise und unter Bezug auf die beigefügten Figuren näher erläutert.

Dabei zeigen:
- Fig. 1: eine erfindungsgemässe Dosiervorrichtung, bestehend aus zwei ineinander drehbare Vorrichtungsteile,
- Fig. 2: in Perspektive von der Seite eine zusammengefügte erfindungsgemässe Dosiervorrichtung,
- Fig. 3a, 3b: in Perspektive die beiden Vorrichtungsteile voneinander getrennt,
- Fig. 4a, 4b: die erfindungsgemässe Vorrichtung in perspektivischem Schnitt, in zusammengefügtem Zustand, verschlossen sowie für den Durchfluss einer Flüssigkeit geöffnet,
- Fig. 5: in Perspektive eine erfindungsgemässe Dosiervorrichtung, angeschlossen an einen Katheter bzw. Infusionsschlauch, und
- Fig. 6a, 6b: eine weitere Ausführungsvariante einer erfindungsgemässen zweiteiligen Vorrichtung im Schnitt.

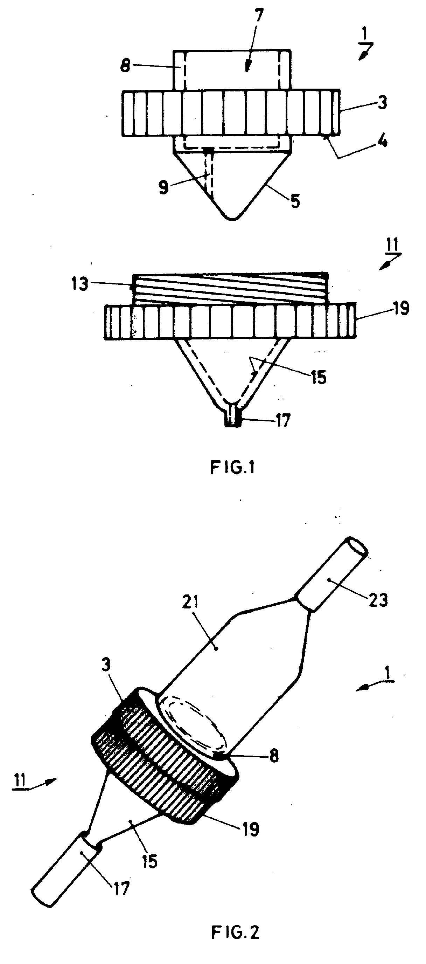

In Figur 1 ist im Schnitt schematisch eine erfindungsgemässe Präzisionsdosiervorrichtung bzw. ein Dosimeter dargestellt, bestehend aus den beiden ineinander schraubbaren Teile 1 und 11. Das eine Teil 1 weist ein Kegeloberteil 5 auf, aufweisend eine kegelförmig ausgebildete Oberfläche. Durch das Kegeloberteil 5 hindurch ausgebildet ist ein Durchgang 9, vorgesehen für den Durchfluss des fluiden Mediums. Weiter weist das eine Teil 1 einen kragenförmig ausgebildeten Rand 3 auf mit innen ausgebildetem Schraubgewinde 4. Schliesslich vorgesehen ist ein kreisrunder Kragen 8, vorgesehen um mit beispielsweise einer Tropfenkammer verbunden zu werden, auf welche später Bezug genommen wird. Innen liegend im Kragen 8 und oberhalb des Kegeloberteiles 5 ausgebildet ist ein Innenraum 7, vorgesehen für die Aufnahme des fluiden Mediums.

Das andere Teil 11 weist eine hohlkegelförmige innere Oberfläche eines Kegelunterteiles 15 auf, vorgesehen für die Aufnahme des Kegeloberteiles 5. In der Spitze des Hohlkegels ist ein weiterer Durchgang sowie ein Anschluss 17 vorgesehen, um das andere Teil bzw. die Dosiervorrichtung, beispielsweise mit einem Infusionsschlauch, zu verbinden. Für das einschraubbare Verbinden des anderen Teiles 11 in das innen liegende Gewinde 4 im einen Teil 1 ist am anderen Teil ein Vorsprung 13 vorgesehen, peripher aufweisend ein entsprechendes Gewinde. Für das Einschrauben des unteren Teiles 11 in das obere Teil 1 ist weiter eine Griffpartie 19 vorgesehen.

Figur 2 zeigt die Dosiervorrichtung in Perspektive in zusammengesetztem Zustand. Dabei sind die beiden Teile 1 und 11 zusammengeschraubt dargestellt, sowie auf den Kragen aufgesetzt ein Tropfkammerzufluss 21, aufweisend einen Anschluss 23 um einen Schlauch am Tropfkammerzufluss anzuschliessen.

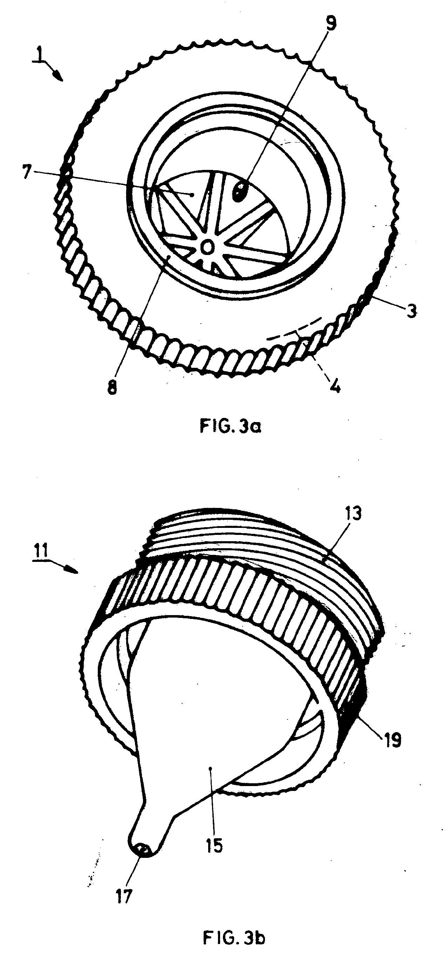

Figur 3 zeigt wiederum in Perspektive die beiden Teile aus Figur 1, wobei Figur 3a das obere eine Teil 1 in Ansicht von oben zeigt, und Figur 3b das untere, andere Teil 11 in Perspektive von unten gesehen. Deutlich erkennbar in Figur 3a ist der innere Aufnahmeraum 7 sowie der Durchgang 9 für den Durchfluss des fluiden Mediums.

Figur 3b zeigt den Drehgriff 19 und das oben am anderen Teil vorstehende Gewinde 13, vorgesehen um in das Innengewinde 4 (in Figur 3a nicht erkennbar) des einen oberen Teiles 1 eingeschraubt zu werden.

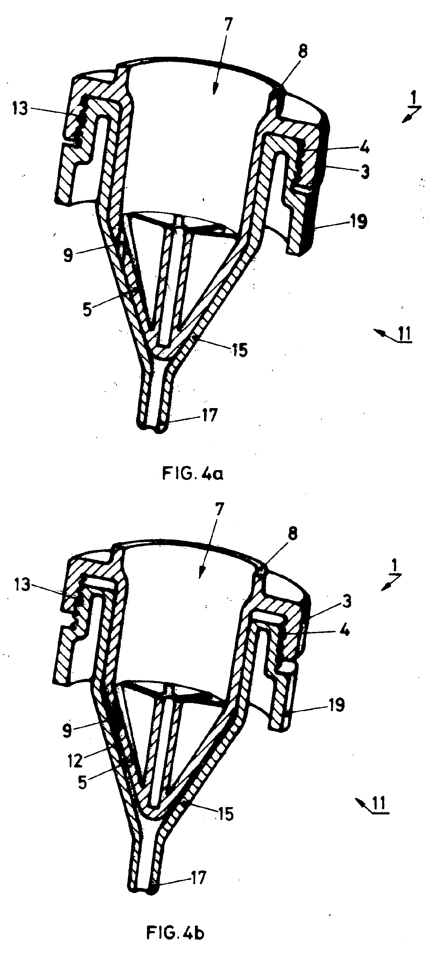

In den Figuren 4a und 4b soll schematisch die Funktionsweise des erfindungsgemässen Dosimeters näher erläutert werden.

Figur 4a zeigt die beiden Teile 1 und 11 im zusammengeschraubten und das Dosimeter im verschlossenen Zustand. Erkennbar ist, wie das Gewinde 13 des anderen Teiles 11 im Innengewinde 4 des einen Teiles 1 eingeschraubt ist. Dabei sind die beiden Teile derart zusammengeschraubt, dass zwischen dem oberen Kegel 5 und dem unteren Hohlkegel 15 kein Abstand vorhanden ist bzw. kein Spalt offen ist. Somit ist der Durchgang 9 im Kegel 5 verschlossen. Durch Drehen des Gewindes 13 im Innengewinde 4 wird die Dosiervorrichtung geöffnet, wie in Figur 4b schematisch dargestellt. Deutlich erkennbar ist nun ein Zwischenraum bzw. ein Spalt 12 zwischen der Kegeloberfläche des oberen Kegels 5 und der Oberfläche des unteren Hohlkegels 15. Somit kann Flüssigkeit, wie beispielsweise eine Infusionsflüssigkeit, vom Aufnahmeraum 7, durch den Durchgang 9 in den Spalt 12 gelangen und von diesem in einen Katheterschlauch, aufgesteckt auf dem Anschluss 17.

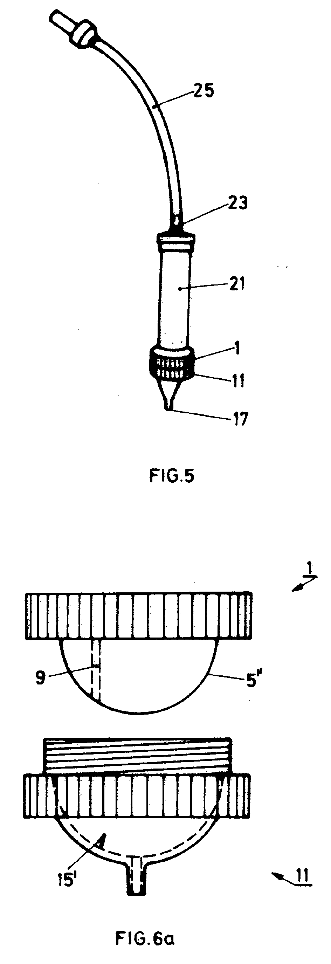

In Figur 5 ist die ganze Dosiervorrichtung schematisch dargestellt, wie sie in einem Infusionsschlauch bzw. Katheter für die Dosierung einer Infusionsflüssigkeit angeordnet ist. Durch eine Zufuhrleitung 25 wird die Infusionsflüssigkeit über eine Tropfkammer 21 der Dosiereinrichtung bzw. dem Dosimeter zugeführt. Durch Schrauben der beiden Teile 1 und 11 ineinander bzw. auseinander kann die Dosierung eingestellt und in der Tropfkammer visuell überprüft werden. Über den Abfluss 17 wird die Infusionsflüssigkeit, beispielsweise einem Patienten, zugeführt.

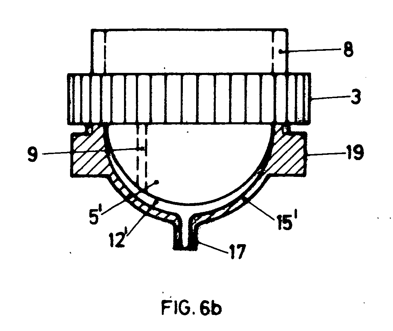

In den Figuren 6a und 6b ist schematisch im Schnitt eine weitere Ausführungsvariante eines erfindungsgemässen Dosimeters dargestellt. Im Unterschied zum Dosimeter, dargestellt in den Figuren 1 bis 5 weisen die beiden Teile keinen Kegelkörper bzw. Hohlkegel auf, sondern am einen Teil ist eine halbkugelartige Oberfläche 5' ausgebildet durch welche hindurch sich der Durchfluss 9 erstreckt, und entsprechend weist das andere Teil eine halbhohlkugelförmige Oberfläche 15' auf. Die übrigen Teile sind analog denjenigen dargestellt im Dosimeter gemäss den Figuren 1 bis 5.

Die Funktionsweise ist analog, indem mittels eines Schraubgewindes die beiden Teile ineinander bzw. auseinander schraubbar sind, um den Durchfluss, beispielsweise einer Infusionsflüssigkeit, zu dosieren.

Beim Dosimeter bzw. der Dosiervorrichtung, dargestellt in den Figuren 1 bis 6, handelt es sich selbstverständlich nur um Beispiele für die bessere Erläuterung der vorliegenden Erfindung. So ist es selbstverständlich möglich, die Oberflächen bzw. die Körperteile der beiden Dosimeterteile andersartig auszugestalten. Wesentlich ist, dass durch Bewegen der beiden Teile zueinander bzw. voneinander eine Dosierung der Flüssigkeit ermöglicht wird. Auch muss nicht zwingend eine Schraubverbindung verwendet werden, sondern es ist auch möglich, die beiden Teile steckbar zueinander bzw. voneinander zu bewegen. Grundsätzlich sind alle Dreh-, Stoss- oder anderen Bewegungsarten möglich, welche eine Veränderung des Querschnittes bzw. des Spaltes und damit des Durchflusses bewirken können.

Auch die für die Herstellung eines Dosimeters verwendeten Materialien sind vielfältig und richten sich primär nach den zu dosierenden Flüssigkeiten. Vorzugsweise werden Polymermaterialien verwendet, welche in den zu spritzenden Dimensionen transparent bzw. weitgehendst durchsichtig sind. Alle üblicherweise in der Medizinaltechnik verwendeten Polymere sind, insbesondere geeignet für die Herstellung des erfindungsgemäss beschriebenen Dosimeters. Falls dickere Wandungen für die Herstellung des Dosimeters zu wählen sind werden vorzugsweise transparente Polymere, wie beispielsweise Acrylglas, Polycarbonat, etc. verwendet, andernfalls ist auch die Verwendung von Polyethylen, Polyamid, etc. möglich. Allerdings ist die Verwendung eines transparenten Polymers insbesondere wesentlich für die Herstellung der Tropfkammer bzw. des Tropfenzählers, währenddem für die beiden ineinander schraubbaren Teile bzw. ineinander steckbaren Teile auch nicht transparente Polymere verwendet werden können. Hierzu ist gar die Verwendung von eingefärbten Polymeren möglich.

Weiter ist es möglich, zusammen mit dem erfindungsgemäss beschriebenen Dosimeter beispielsweise eine Schiebeklemme zu verwenden, um beispielsweise kurzzeitige Infusionsunterbrechung bzw. eine kurzzeitige Unterbrechung des Durchflusses zu ermöglichen. Weiter kann es sinnvoll sein, ein Filtersystem im erfindungsgemäss beschriebenen Dosimeter zu integrieren, um beispielsweise mechanische Verunreinigungen zurückzuhalten.

Selbstverständlich ist die erfindungsgemäss beschriebene Dosiervorrichtung keinesfalls auf die Verwendung bei Infusionen beschränkt, sondern die erfindungsgemässe Vorrichtung kann auch für die Dosierung irgendwelcher anderer fluider Medien, wie insbesondere Flüssigkeiten, verwendet werden. Auch bei chemischen Apparaten, in der Reaktionstechnik, der Medizinaltechnik, in der Lebensmitteltechnik, etc., etc., können die erfindungsgemäss beschriebenen Dosiereinrichtungen verwendet werden.

## Patentansprüche

1. Präzisionsdosiervorrichtung für fluide Medien, **gekennzeichnet durch** mindestens zwei in Richtung zueinander bzw. voneinander bewegbare Teile (1, 11), wobei die beiden Teile je einen flächigen Abschnitt (5, 15) aufweisen, welche voneinander beabstandet benachbart zueinander angeordnet sind, derart, dass beim Bewegen der beiden Teile der Abstand (12) zwischen den beiden flächigen Abschnitten verkleinerbar bzw. vergrösserbar ist, und dass der Durchfluss des fluiden Mediums zwischen mindestens an Teilbereichen der beiden Abschnitten bzw. Flächen regulierbar bzw. dosierbar ist.

2. Vorrichtung nach Anspruch 1, **dadurch gekennzeichnet, dass** die beiden Abschnitten bzw. Flächen (5, 15) wenigstens entlang eines Teilbereiches, wenigstens nahezu formschlüssig zueinander passend, ausgebildet sind.

3. Vorrichtung nach einem der Ansprüche 1 oder 2, **dadurch gekennzeichnet, dass** die beiden Teile (1, 11) mittels eines Schraubgewindes voneinander bzw. zueinander bewegbar sind.

4. Vorrichtung nach einem der Ansprüche 1 bis 3, **dadurch gekennzeichnet, dass** das eine Teil (1) bzw. dessen Oberfläche (5) kegelförmig ausgebildet ist und das andere Teil (11) bzw. dessen Oberfläche (15) wenigstens nahezu dazu entsprechend hohlkegelförmig ausgebildet ist, und dass im oder am einen Teil aufweisend die kegelförmige Oberfläche mindestens ein Durchgang (9) für das fluide Medium vorgesehen ist, damit das Medium in den Zwischenraum (12) zwischen die beiden flächigen Abschnitte (5, 15) einfliessen kann.

5. Vorrichtung nach einem der Ansprüche 1 bis 4, **dadurch gekennzeichnet, dass** am einen Teil (11) eine Zuflusstropfkammer (21) vorgesehen ist.

6. Vorrichtung nach einem der Ansprüche 1 bis 5, dass am anderen Teil (11) aufweisend beispielsweise eine hohlkörperförmige Oberfläche (15), ein Anschluss (17) vorgesehen ist für das Anschliessen, beispielsweise eines Schlauches.

7. Verfahren zum Dosieren eines fluiden Mediums, beispielsweise bei Infusionen und dgl., **dadurch gekennzeichnet, dass** das fluide Medium in einem Zwischenraum zwischen zwei voneinander beabstandete flächige Abschnitte von je einem Teil einer Dosiervorrichtung eingebracht wird, und dass durch Bewegen der beiden Teile zueinander bzw. voneinander der Zwischenraum bzw. der Abstand der beiden flächigen Abschnitte verkleinert bzw. vergrössert wird, um das fluide Medium zu dosieren.

8. Verfahren nach Anspruch 7, **dadurch gekennzeichnet, dass** das fluide Medium durch einen Durchgang in einen Teil bzw. der Oberfläche des einen Teils in den Zwischenraum eingegeben wird und durch Bewegen der beiden flächigen Abschnitte bzw. Oberflächen, wie beispielsweise mittels eines Gewindes, voneinander bzw. zueinander die gewünschte Dosierung eingestellt wird, indem der Abstand bzw. Zwischenraum zwischen den Oberflächen verkleinert bzw. vergrössert wird, und anschliessend das Medium durch einen weiteren Durchgang im anderen Teil bzw. in der anderen Oberfläche den Zwischenraum in gewünschter Dosierung verlässt.

9. Verfahren nach einem der Ansprüche 7 oder 8, **dadurch gekennzeichnet, dass** zunächst das fluide Medium durch eine Tropfkammer geführt wird, um dann durch einen Durchgang in einer beispielsweise kegelförmig ausgebildeten Oberfläche in den Zwischenraum geführt zu werden, welcher durch die kegelförmige Oberfläche und eine hohlkegelförmig ausgebildete Oberfläche gebildet wird; durch Bewegen der beiden Teile, wie durch eine Drehbewegung, mittels eines Gewindes die beiden Oberflächen, wie der Kegel und der dazu weitgehendst formschlüssig ausgebildete Hohlkegel zueinander bzw. voneinander bewegt werden um die gewünschte Dosierung, beispielsweise in der Tropfkammer, visuell überprüfbar einzustellen, und dass das Medium durch eine Öffnung, beispielsweise angeordnet in der Spitze des hohlkegelförmigen Körpers, den Zwischenraum in gewünschter Dosierung verlässt, um beispielsweise einem Schlauch, wie einem Infusionsschlauch, zugeführt zu werden.

10. Verwendung der Dosiervorrichtung nach einem der Ansprüche 1 bis 6 zum Dosieren in der Medizinaltechnik, wie insbesondere von Infusionen bzw. Infusionslösungen.

11. Verwendung der Dosiervorrichtung zum Dosieren von Reagenzien bei der Durchführung von chemischen Reaktionen.

12. Verwendung der Dosiervorrichtung in der Lebensmittelindustrie.
